Europäisches Patentamt

European Patent Office

Office européen des brevets

(11) Veröffentlichungsnummer: **0 315 757**
**A2**

## (12) EUROPÄISCHE PATENTANMELDUNG

(21) Anmeldenummer: **88115162.5**

(22) Anmeldetag: **16.09.88**

(51) Int. Cl.4: **G01N 33/49**

(30) Priorität: **12.11.87 CH 4423/87**

(43) Veröffentlichungstag der Anmeldung:
**17.05.89 Patentblatt 89/20**

(84) Benannte Vertragsstaaten:
**AT BE CH DE FR GB IT LI NL SE**

(71) Anmelder: **CONTRAVES AG**
**Schaffhauserstrasse 580**
**CH-8052 Zürich(CH)**

(72) Erfinder: **Windisch, Arthur**
**Lachenstrasse 43**
**CH-8184 Bachenbülach(CH)**
Erfinder: **Pfenninger, Heinz**
**Etzelweg 15**
**CH-8604 Volketswil(CH)**

(54) **Verfahren und Vorrichtung zum Speichern und Mischen von Blutproben.**

(57) Die Vorrichtung zum Speichern und Mischen von Blutproben weist eine Speicher- und Mischeinheit (2) auf, die aus einer Speicherscheibe (15) und einer Mischerscheibe (17) für Blutproben (4) besteht. Die koaxiale Speicherscheibe (15) und die Mischerscheibe (17) sind unabhängig voneinander angetrieben und mit zu einander komplementären Nuten (42) und (43) versehen. Eine Ladevorrichtung (3) lädt die Blutproben (4) nacheinander in die Speicherscheibe (15), die sie nach einer Uebergabevorrichtung (5) transportiert. Die Übergabevorrichtung (5) befördert die Blutproben (4) nacheinander in eine gegenüberliegende Nut (43) der Mischerscheibe (17). Die Mischerscheibe (17) dreht die Blutproben (4) während einer bestimmten Mischdauer vor der Uebergabe an eine Blutentnahmevorrichtung (7). Die Blutentnahmevorrichtung (7) versetzt die Blutprobe (4) um ihre Längsachse in Drehung, so dass eine Barcodelesevorrichtung (6) den Barcode (142) auf der Blutprobe unabhängig von deren Lage zuverlässig lesen kann. Danach saugt die Blutentnahmevorrichtung (7) das Blut aus der Blutprobe (4) und leitet es einem Blutanalysator zu. Die Mischerscheibe (17) dreht nun die Blutprobe (4) nach einer Auswerfposition (155), wo die Blutprobe die Mischerscheibe (17) verlässt und in einem der Behälter (8,9,10) für die verschiedenen Probentypen aufgefangen wird.

FIG. 1

# VERFAHREN UND VORRICHTUNG ZUM SPEICHERN UND MISCHEN VON BLUTPROBEN

Die Erfindung betrifft eine Vorrichtung zum Speichern und Mischen von Blutproben gemäss Oberbegriff des Patentanspruchs 1, sowie Verfahren dafür.

In der automatisierten Blutanalyse müssen von einem bestimmten Automatisierungsgrad an die Blutproben dem Analyseautomaten maschinell zugeführt werden. Dabei ist darauf zu achten, dass Blut ein sehr empfindliches Material ist, so dass es nur mit Sorgfalt manipuliert werden darf. So darf das zu analysierende Vollblut nicht über längere Zeit stehen, da sonst Sedimentation auftritt. Es darf auch nicht brüsk bewegt, z.B. geschüttelt werden, da dann die empfindlichen Blutzellen geschädigt werden. Da es bekannt ist, dass insbesondere Vollblut sehr schonend gehandhabt werden muss, sind dazu entsprechende Geräte entwickelt worden, insbesondere Geräte zum Mischen, oder besser gesagt, zum Bewegen des Blutes, bevor es zur Analyse gelangt. Es sind jedoch in der Regel weder die Bewegungsabläufe beim Mischen noch die dazu entwickelten Geräte geeignet, um in eine Automatisierungskette integriert werden zu können. Dies liegt an den dazu erforderlichen Uebergangsstellen, z.B. vom Mischen bis zum Entnehmen der Blutprobe. Solche Uebergangsstellen sind nicht selten mechanische Kompromisse und befriedigen weder von der Bearbeitungsgeschwindigkeit noch von der Bearbeitungssicherheit.

Der Erfindung liegt die Aufgabe zugrunde eine Vorrichtung zum Speichern und Mischen von Blutproben zu schaffen, die eine Blutprobe annimmt, bis zur Analyse adäquat bereithält und dann dem Analyseautomaten das zu untersuchende Blut zur Analyse zuführt. Die Vorrichtung soll jeder Anpassung an die Arbeitsgeschwindigkeit des Automaten genügen und die Bearbeitungssicherheit soll nicht nur technisch, sondern auch ablaufmässig sicher sein, so dass Fehlanalysen minimiert bzw. eliminiert werden. Ferner ist es Aufgabe der Erfindung, eine Vorrichtung so auszugestalten, dass damit in einem Behälter verschlossene Blutproben ohne zu Oeffnen verarbeitet werden können.

Diese Aufgabe wird durch eine Vorrichtung zum Vorbereiten von Blutproben zur automatischen Analyse gelöst, die sich dadurch kennzeichnet, dass eine Speicherscheibe zur Speicherung der Blutproben und eine, unabhängig davon angetriebenen Mischerscheibe zur Vorbereitung dieser Blutproben vorgesehen sind, wobei die Scheiben nebeneinander angeordnet auf der einander zugekehrten Seite mit radialen, einander komplementären Nuten zum Aufnehmen einer Blutprobe versehen sind, sowie durch eine Uebergabevorrichtung zum Uebergeben von Blutproben von der Speicherscheibe an die Mischerscheibe. Die Unteransprüche betreffen vorteilhafte Weiterbildungen.

Ein Ausführungsbeispiel des Erfindungsgegenstandes ist nachfolgend anhand der Zeichnungen beschrieben. Es ist:

Fig.1 eine Seitenansicht der Vorrichtung gemäss der Erfindung

Fig.2 der Querschnitt 2-2 in Fig.1

Fig.3 der Querschnitt 3-3 in Fig.2

Fig.4 die Teildraufsicht 4-4 in Fig.2

Fig.5 die Ansicht 5-5 in Fig.2

Fig.6 die Ansicht 6-6 in Fig.2

Fig.7 die Ladevorrichtung in Draufsicht

Fig.8 der Querschnitt 8-8 in Fig.7

Fig.9 ein Querschnitt der Blutentnahmevorrichtung

Fig.10 die Ansicht 10-10 in Fig.9

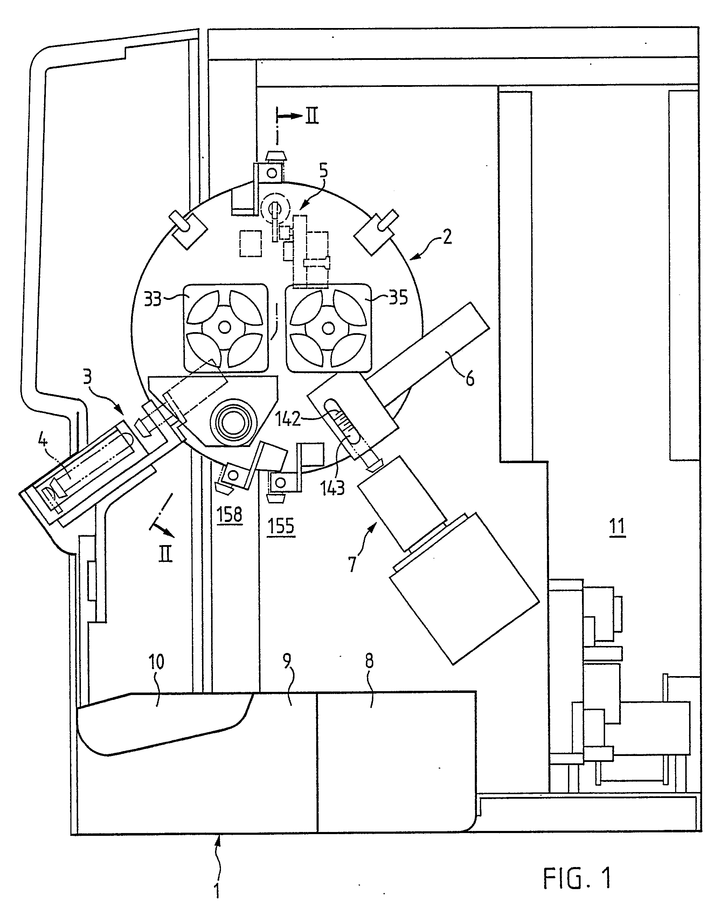

Nach Fig.1 enthält ein Gehäuse 1 eine Speicher- und Mischeinheit 2 für Blutproben, eine Ladevorrichtung 3 für Blutproben 4, eine Uebergangsvorrichtung 5 für Blutproben, eine Barcode-Lesevorrichtung 6 zur Patienten-Identifizierung der Blutproben, eine Blutentnahmevorrichtung 7 zum Entnehmen von Blut für die Analyse aus den Blutproben(behälter), einen Aufnahmebehälter 8 für ordnungsgemäss bearbeitete und identifizierte Blutproben, einen Aufnahmebehälter 9 für nicht identifizierbare Blutproben, einen Aufnahmebehälter 10 für Notfall-Blutproben und einen Raum 11 mit der Steuerelektronik und der Verfahrens-Software zum Steuern der verschiedenen Funktionen der Vorrichtung.

Die Speicher- und Mischeinheit 2 für die Blutproben kann, bezogen auf die Unterlage oder die Richtung der Schwerkraft, vertikal (parallel) oder geneigt angeordnet sein. Bei einer vertikalen Anordnung (parallel zur Schwerkraft), was insbesondere bei der Mischerscheibe von Einfluss ist, werden die Blutproben über Kopf gemischt; bei einer Ausführungsform mit geneigten Scheiben, ist der Mischvorgang näher dem der bekannten Vollblut-Probenmischer. Die Neigung kann 1° bis 89° betragen, wobei Neigungswinkel zur Vertikalen zwischen 30° und 60° die effizientesten sind, vorzugsweise wird ein Neigungswinkel von 45° vorgeschlagen. Eine vorgegebenen Lageabhängigkeit der Speicher- und Mischeinheit 2 ist jedoch nicht Sachzwang sondern freie Entscheidung. Die nachfolgend diskutierte beispielsweise Ausführungsform ist diejenige mit vertikal zur Unterlage bzw. parallel zur Schwerkraft angeordneten und arbeitenden Scheiben. Doch jede geneigte Anordnung funktioniert mechanisch gleichermassen, so dass keine speziellen konstruktiven Massnahmen an den wesentlichen Vorrichtungseinheiten zu treffen sind, als eben die der Schrägstellung der Scheiben. Dies ist auch mit ein Grund, warum eine Ausführungsform mit "Schrägstellung" nicht detailliert beschrieben werden muss.

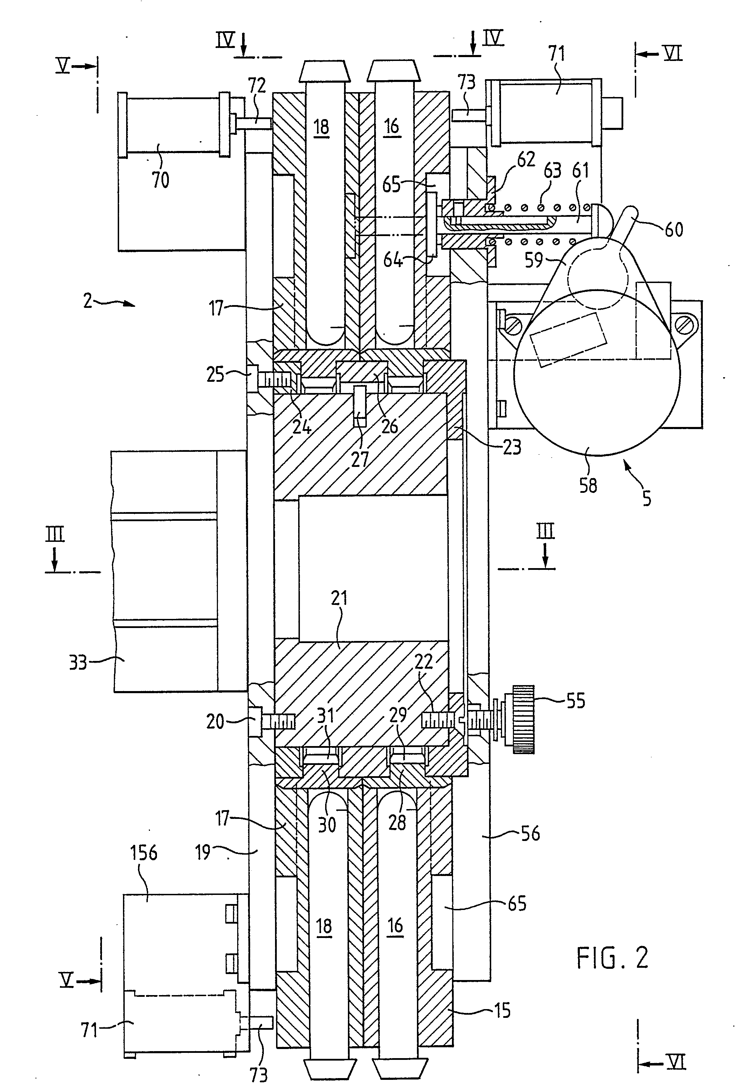

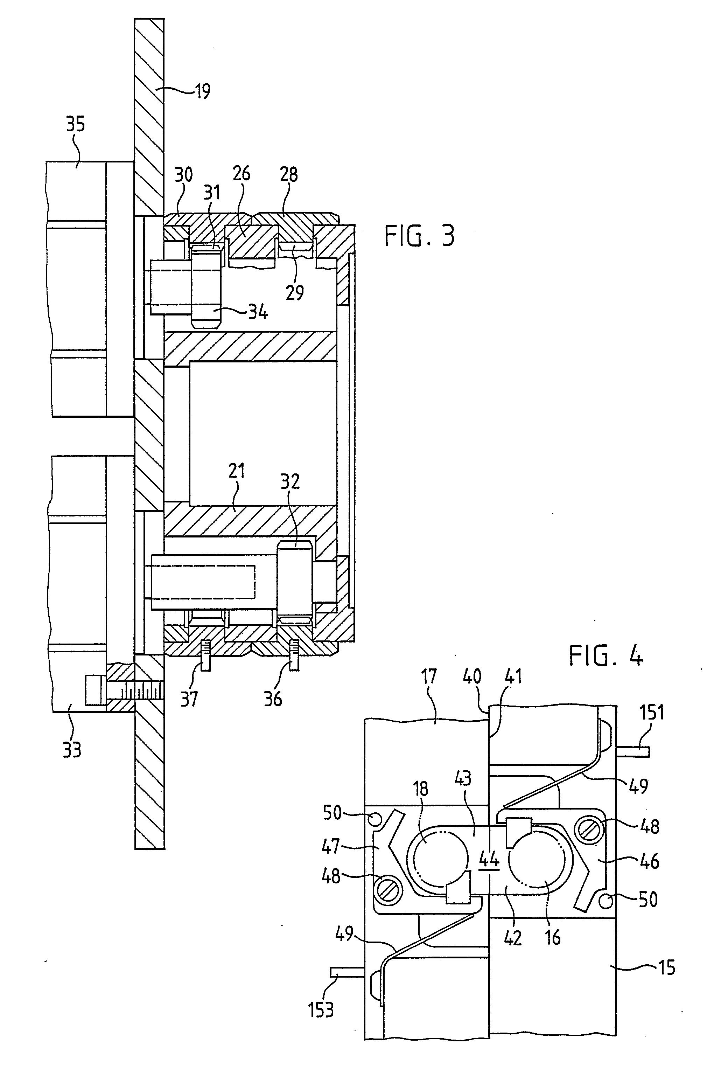

Wie aus Fig. 2 ersichtlich, besteht die Speicher- und Mischeinheit 2 aus einer Speicherscheibe 15 für Blutproben 16 und einer Mischerscheibe 17 für Blutproben 18, die wie folgt separat angetrieben werden. Auf einer kreisförmigen Platte 19 ist mit Schrauben 20 eine Nabe 21 befestigt. Auf der Nabe 21 ist mit Schrauben 22 ein Ring 23 befestigt. Ein weiterer Ring 24 auf der Nabe 21 ist mit Schrauben 25 ein Ring 19 befestigt. Die Nabe 21 trägt weiterhin einen dritten Ring 26, der mittels eines Stiftes 27 gegen Drehen gesichert ist. Auf dem Ring 23 und der einen Hälfte des Ringes 26 ist ein Zahnrad 28 mit einem inneren Zahnkranz 29 frei drehbar gelagert. Auf dem Ring 24 und der anderen Hälfte des Ringes 26 ist ein Zahnrad 30 mit einem inneren Zahnkranz 31 frei drehbar gelagert. In den Innenkranz 29 des Zahnrades 28 (Fig.3) greift ein Antriebsritzel 32 eines Motors 33 (bspw. ein Schrittmotor), der auf der Platte 19 befestigt ist, ein. In den Innenkranz 31 des Zahnrades 30 greift ein Antriebsritzel 34 eines weiteren Motors 35, der ebenfalls auf der Platte 19 befestigt ist, ein. Auf dem Zahnrad 28 ist die Speicherscheibe 15 gelagert und mittels eines Mitnehmers 36 damit verbunden. Auf dem Zahnrad 30 ist die Mischerscheibe 17 gelagert und mittels eines Mitnehmers 37 damit verbunden.

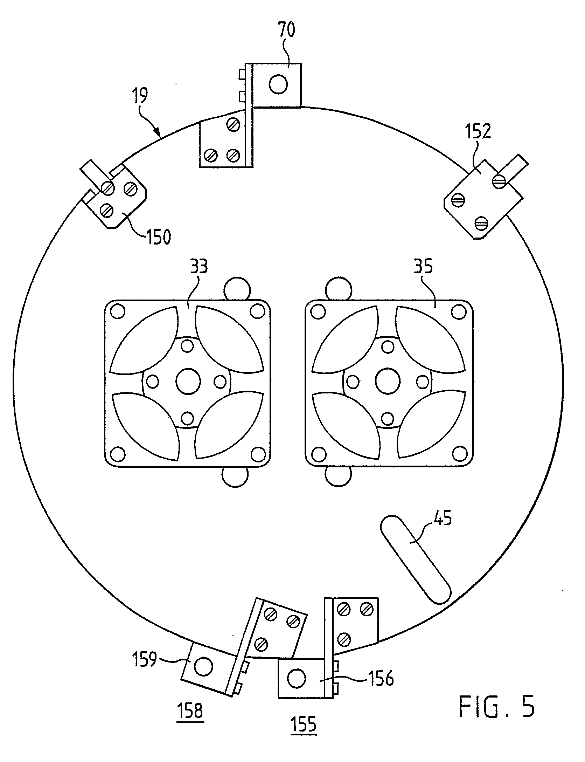

Beide Scheiben, die Speicherscheibe 15 und die Mischerscheibe 17, sind in gleichen, regelmässigen Bogenabständen auf der einander zugekehrten Seite 40 bzw. 41 (Fig.4) mit radialen, einander gegenüberliegenden Nuten 42 bzw. 43 versehen, so dass je zwei Nuten komplementär einen gemeinsamen, am Umfang der Scheiben offenen Hohlraum 44 bilden. Jede Nut kann eine Blutprobe aufnehmen.Beide Scheiben sind jeweils dort, wo sich eine Nut befindet, mit einem radialen Fenster 45 (Fig.5) von ungefähr der Länge der Nut versehen. Für jede Nut beider Scheiben 15 bzw. 17 (Fig.4) ist am Umfang eine Klemme 46 bzw. 47 angebracht, die zum Festhalten der darin angebrachten Blutprobe dient. Die Klemme 46 hat die Form eines Kniehebels und ist um eine Schraube 48 drehbar und wird durch eine Blattfeder 49 in die Nut 42 gedreht bis sie durch einen Anschlag 50 am Weiterdrehen gehindert wird. Die Klemmen 47 sind in derselben Weise gebaut. Vorzugsweise haben beide Scheiben den gleichen Durchmesser und sind koaxial zueinander angeordnet, jedoch sind andere Ausführungsformen möglich, deren Scheiben einen unterschiedlichen Durchmesser, sowie daraus unterschiedliche Nutenzahlen aufweisen und natürlich nicht mehr koaxial zueinander angeordnet sind. Wichtig ist jedoch, dass an einer Uebergabestelle für beide Scheiben eine Kommunikationsbedingung eingehalten werden muss, nämlich dass: Nuten beider Scheiben sich zur gleichen Zeit gegenüber stehen.

Auf dem Ring 23 (Fig.2) ist mit schnellösbaren Schrauben 55 eine Platte 56 befestigt, welche die Uebergabevorrichtung 5 trägt. Die Uebergabevorrichtung besitzt zum Antrieb bspw. einen Synchronmotor 58, der über ein Getriebe 59 kontinuierlich eine Kurbel 60 im Uhrzeigersinn dreht. Die Kurbel 60 wirkt auf eine Schubstange 61, die in einer Führung 62 hin und her gleiten kann und unter dem auswärts gerichteten Druck einer Feder 63 steht. Die Schubstange 61 ist an ihrem anderen Ende mit einer Scheibe 64 versehen, die sich in der Ausgangslage der Kurbel 60 in einer Ringnut 65 der Speicherscheibe 15 befindet. Bei jeder Umdrehung der Kurbel 60 befördert die Scheibe 64 eine Blutproben, z.B. Blutprobe 16, aus der Nut 42 in der Speicherscheibe 15 hinüber in die gegenüberliegende, leere Nut 43 in der Mischerscheibe 17. Beide Scheiben stehen während der Uebergabe der Blutprobe still. Bevor die Uebergabe der Blutprobe stattfinden kann, müssen die Klemmen 46 und 47 geöffnet werden. Dazu ist auf der Platte 19 und der Platte 56 je ein Elektromagnet 70 bzw. 71 mit einem Anker 72 bzw. 73 befestigt. Bei Erregung der Elektromagneten werden die Anker 72 bzw. 73 gegen die Klemmen 46 bzw. 47 gedrückt und drehen sie gegen den Druck der Blattfeder 49 und 49' weg von der Blutprobe, so dass diese die Nut verlassen und in die gegenüberliegende Nut 43 übertreten kann. Nach dem Uebergeben werden die Elektromagneten wieder stromlos gemacht und die Klemmen schliessen sich wieder.

Jede Scheibe erfüllt ihre eigene Aufgabe. Die Speicherscheibe dient zur Aufnahme der Blutproben aus der Ladevorrichtung und befördert diese zur Uebergabestelle, und zwar immer dann, wenn in der Mischerscheibe eine verfügbare freie Position vorhanden ist. Ansonsten steht sie bevorzugtermassen still, um die Vollblutproben nicht unnötigen mechanischen Belastungen auszusetzen. Die Mischerscheibe dagegen, wird bevorzugt in Bewegung gehalten und hat die Aufgabe, das Blut vor der Blutentnahme schonend zu mischen. Sie wird in ihrer Bewegung nur dann angehalten, wenn eine Blutprobe entnommen oder ausgestossen wird oder wenn eine Blutprobe von der Speicherscheibe übernommen wird.

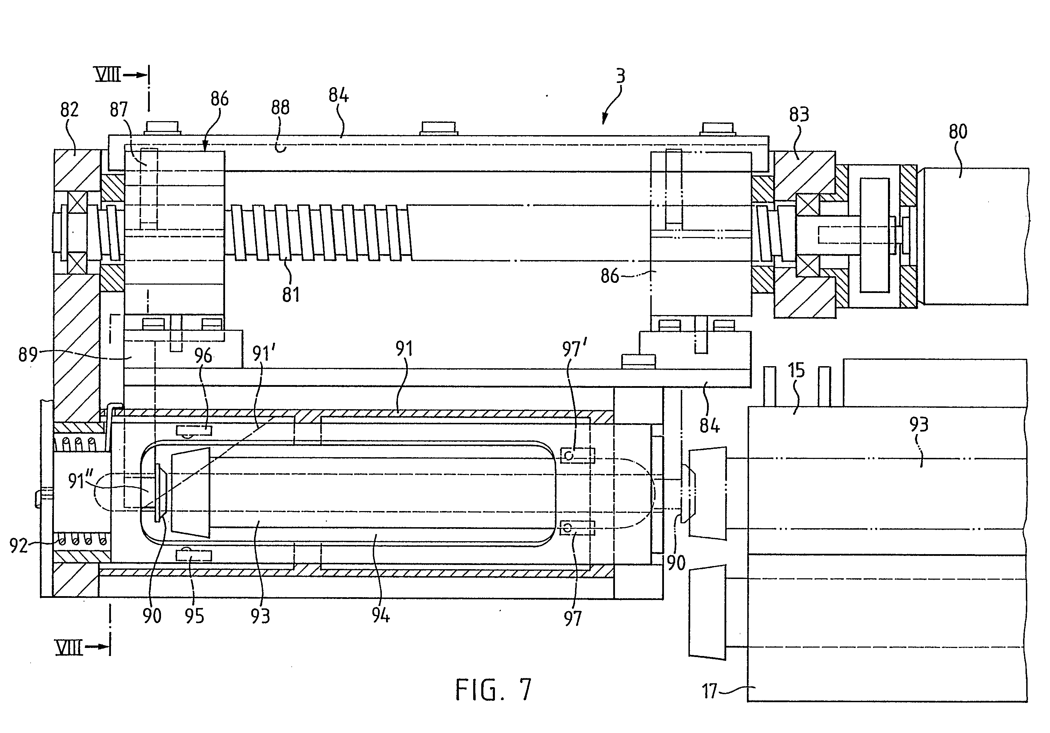

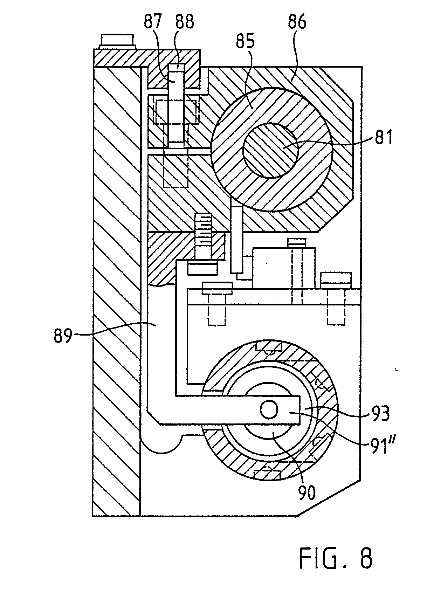

Die Ladevorrichtung 3, die radial auf die Speicherscheibe 15 ausgerichtet ist, dient dazu, jeweils vom Bediener in die Vorrichtung gelegte Blutproben in eine Nut der Speicherscheibe zu befördern. Ein Motor 80 (Fig.7 und 8) treibt eine Gewindespindel 81 an, die in zwei Lagern 82 und 83 in einem Rahmen 84 gelagert ist. Auf der Schraubenspindel 81 befindet sich eine Mutter 85, die in einer Klemme 86 befestigt ist. Die Klemme 86 trägt einen Stift 87, der in einer Nut 88 am Rahmen 84 geführt ist, zwecks Verhinderung der Drehung der Mutter 85 bei der Drehung der Schraubenspindel 81. Auf der Klemme 86 ist ein Mitnehmer 89 befestigt, der mit einer Führungsscheibe 90 versehen ist. Die Ladevorrichtung ist wegen einer möglichen Verletzungsgefahr immer dann gegen aussen durch eine Schirmkappe 91 vollständig abgeschlossen, wenn nicht gerade eine Blutprobe eingelegt wird. Zum Einlegen der Blutprobe 93 wird die Führungsscheibe 90 mit dem Motor 80 ausgefahren, das heisst, von der Speicherscheibe 15 wegbewegt. Damit wird der Raum 94 zum Einlegen einer Blutprobe geschaffen. Dabei gleitet die schräge Kante 91′ auf einem Mitnehmer 91″, wodurch die Schirmkappe gegen die Spannung der Torsionsfeder 92 auf den letzten 10mm des Ausfahrwegs geöffnet wird. Nun kann die Blutprobe 93 in den offenen Eingabeschacht eingelegt werden.

Das Vorhandensein einer Blutprobe 93 im Eingabeschacht wird durch die Gabel-Lichtschranken 95,96 und 97,97′ der elektronischen Steuerung gemeldet, welche daraufhin die richtige Positionierung der Speicherscheibe vornimmt. Danach wird der Motor 80 für den Antrieb der Schraubenspindel 81 eingeschaltet. Die Schraubenspindel setzt die Mutter 85 in Bewegung, die über den Mitnehmer 89 und die Führungsscheibe 90 die Blutprobe in die korrespondierende Nut (das ist die Aufnahmeöffnung) der Speicherscheibe 15 einschiebt. Während den ersten 10mm des Einschiebeweges wird über die schräge Kante 91′ und den Mitnehmer 91″ die Schirmkappe 91 mit der Torsionsfeder 92 geschlossen. Sobald die Blutprobe vollständig in die Speicherscheibe eingeschoben ist, wird ein Endschalter betätigt, welcher über die Steuerung den Spindelmotor 80 ausschaltet und den Antriebsmotor 33 für die Speicherscheibe 15 einschaltet.

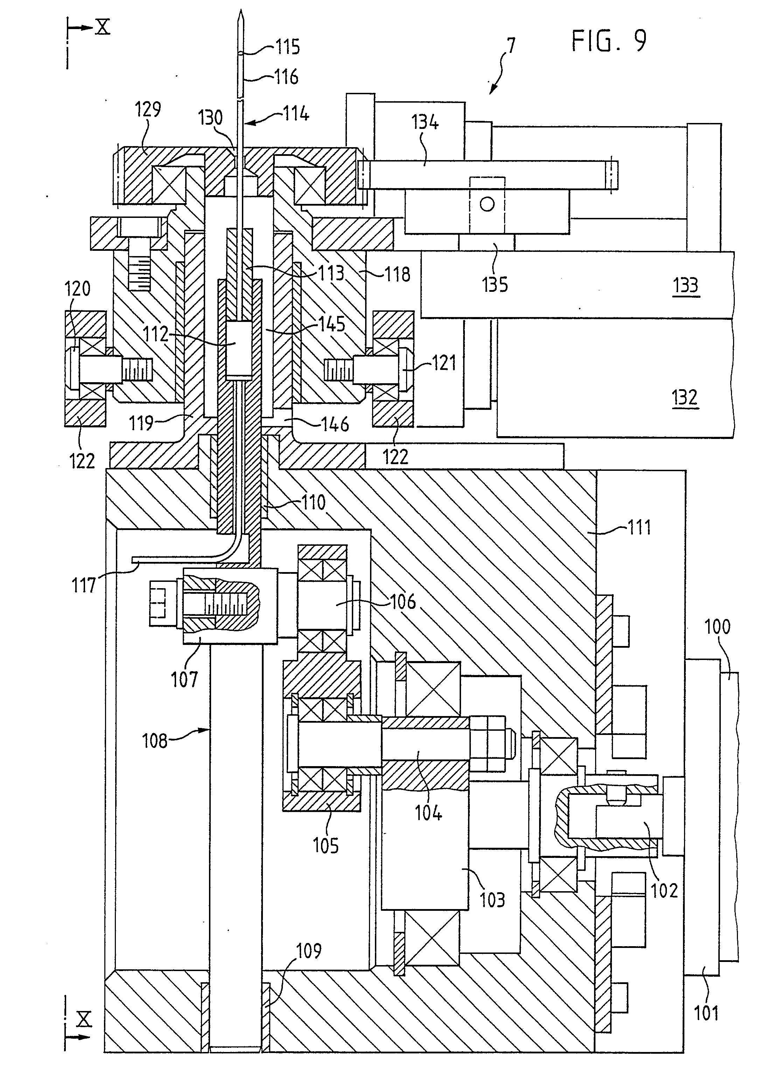

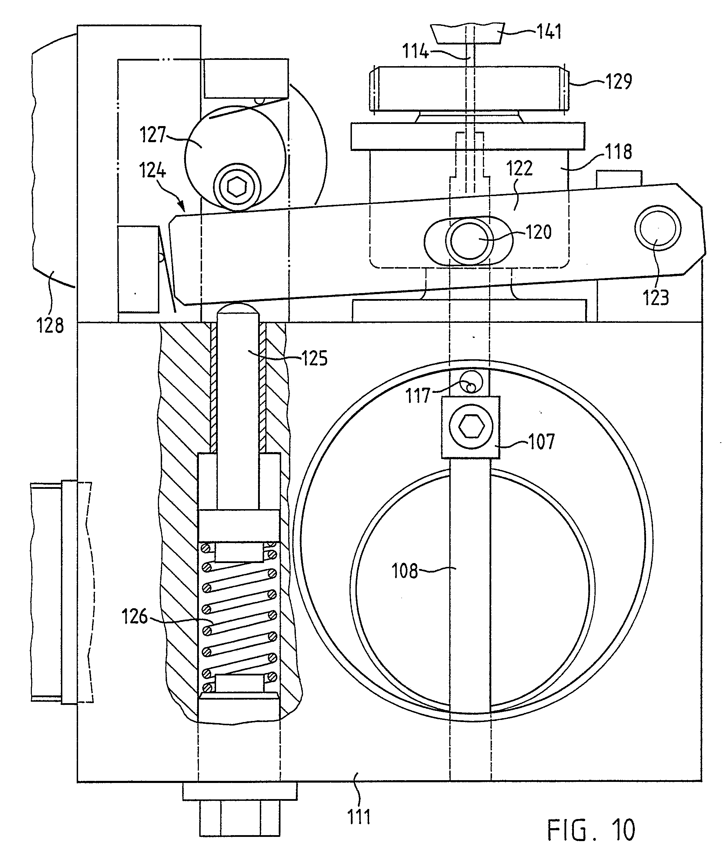

Die Blutentnahmevorrichtung 7, die radial auf die Mischerscheibe 17 ausgerichtet ist, dient dazu, jeweils aus einer Blutprobe Blut zu entnehmen und dieses dem (nicht gezeichneten) nachgeschalteten Blutanalysator zuzuführen. Ein Motor 100 (Fig. 9) treibt über ein Getriebe 101 und dessen Ausgangswelle 102 eine Kurbel 103 mit einem Kurbelzapfen 104 eine Treibstange 105 an. Ein Ende der Treibstange 105 ist um einen Zapfen 106 einer Klemme 107 drehbar. In der Klemme 107 befindet sich ein Nadelträger 108, der in zwei Lagern 109 und 110 eines Gehäuses 111 auf und ab gleiten kann. In einer Bohrung 112 im oberen Ende des Nadelträgers 108 ist mittels einer Klemme 113 eine Hohlnadel 114 befestigt. Die Hohlnadel 114 ist mit einer Oeffnung 115 in der Wand 116 versehen. Die Bohrung 112 im Nadelträger 108 steht über einem Schlauch 117 und über ein (nicht gezeichnetes) Zweiwegventil mit dem Blutanalysator und mit einer Quelle für ein Spülmittel für die Nadel in Verbindung. Der Nadelträger 108 ist konzentrisch von einem umgekehrten Topf 118 umgeben, der auf einem Lager 119 auf und abwärts gleiten kann. Der Topf 118 ist mittels zwei Zapfen 120 (Fig.10) und 121 von einer Gabel 122 getragen, deren eines Ende um eine Achse 123 drehbar ist. Ein freies Ende 124 der Gabel 122 steht einerseits über einen Bolzen 125 unter der Kraftwirkung einer Feder 126, andererseits über einen Exzenter 127 unter der Kraftwirkung eines zweiten Motors 128. Auf dem Topf 118 ist ein Zahnrad 129 frei drehbar gelagert, das eine zentrale Bohrung 130 zum Passierenlassen der Hohlnadel 114 aufweist. Ein dritter Motor 132 treibt über ein Getriebe 133 ein Zahnrad 134 auf dessen Ausgangswelle 135 an, das mit dem Zahnrad 129 auf dem Topf 118 kämmt. Sobald eine Blutprobe 140 vor der Blutentnahmevorrichtung angekommen ist, wird der Motor 128 eingeschaltet und der Exzenter 127 ermöglicht es durch seine Drehung der Feder 126 die Gabel 122 anzuheben, so dass auch der Topf 118 mit dem Zahnrad 129 angehoben wird. Das Zahnrad 129 wird dabei gegen die Gummikappe 141 der Blutprobe 140 in der Mischerscheibe 17 gedrückt, so dass eine Reibungsverbindung zwischen beiden entsteht. Daraufhin wird der Motor 132 eingeschaltet und das Zahnrad 134 in Drehung versetzt, das seinerseits das Zahnrad 129 antreibt, wodurch die Blutprobe 140 in ihrer Nut in der Mischerscheibe 17 um ihre Längsachse gedreht wird. Der Barcode 142 auf der Blutprobe rotiert somit vor dem Fenster 143 in der Mischerscheibe. Gleichzeitig wird die Barcodelesevorrichtung 6 eingeschaltet und diese stellt die Patientenidentität der Blutprobe fest. Der Zweck der Drehung der Blutprobe während des Lesens ist zu gewährleisten, dass die Barcodelesevorrichtung den Code auf der Blutprobe lesen kann, ungeachtet deren Lage in der Nut. Nach dem Lesen des Codes wird der Motor 132 ausgeschaltet, so dass die Blutprobe zum Stillstand kommt. Danach wird der Motor 100 eingeschaltet, so dass der Nadelträger 108 angehoben wird und die Nadel 114 die Gummikappe 141 der Blutprobe 140 durchsticht. Die notwendige Menge von Blut wird aus der Probe gesaugt und über den Schlauch 117 dem Blutanalysator zugeführt. Durch die Weiterdrehung des Motors 100 wird der Nadelträger 108 jetzt abwärts bewegt, so dass die Nadel 114 zurückgezogen wird. Der Motor 128 drückt die Gabel 122 abwärts, so dass das Zahnrad 129 von der Blutprobe freikommt. Durch den Schlauch 117 wird nun ein Spülmittel in die Bohrung 112 des Nadelträgers geleitet, das die Nadel durchströmt und durch die Wandöffnung 115 und einen Ringraum 145 im Lager 119 durch eine Oeffnung 146 abgeführt wird. Die Mischerscheibe wird jetzt wieder eingeschaltet und die nächste Blutprobe vor die Blutentnahmevorrichtung gedreht.

Zusammengefasst arbeitet die Vorrichtung wie folgt:

Eine Blutprobe besteht zum Beispiel aus einem Glasröhrchen, das, anfänglich evakuiert, bei der Blutentnahme mit Blut gefüllt wurde, und mit einer Gummikappe verschlossen ist.Die Speicherscheibe und die Mischerscheibe werden beide durch ihre Motoren im Gegenuhrzeigersinn angetrieben. Die Blutproben werden einzeln von Hand in die Ladevorrichtung gelegt. In der Mischerscheibe wird das Blut durch die Drehung schonend gemischt. Die Speicherscheibe kann unabhängig von der Mischerscheibe mit Blutproben bestückt werden. Beim Einlegen einer Blutprobe wird durch die Steuerung die Speicherscheibe positioniert und der Motor für die Transportspindel eingeschaltet, welche die Blutprobe in die Speicherscheibe befördert. Danach wird der Motor für die Speicherscheibe durch die Steuerung wieder eingeschaltet.

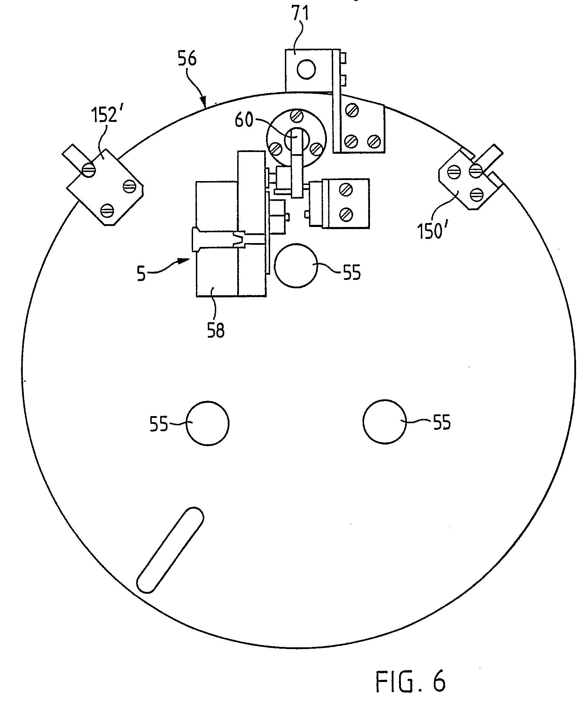

Auf dem Weg zur Uebergabevorrichtung 5 passiert die Speicherscheibe eine Gabel-Lichtschranke 150', welche auf der Platte 56 befestigt ist (Fig. 5 und 6). Das Auslöseelement ist ein für jede Nut auf der Speicherscheibe befestigter Stift 151 (Fig. 4), welcher die Lichtschranke durchläuft. Bei jedem Durchgang erzeugt die Lichtschranke einen Impuls für die Steuerung, die Impulse zählt, speichert und zur Steuerung der Speicherscheibe verwendet. Bei der Uebergabevorrichtung angekommen, werden die Speicherscheibe und die Mischerscheibe angehalten. Die dortigen Elektromagnete 70 und 71 werden erregt und sie öffnen die Klemmen in den betreffenden Nuten beider Scheiben. Gleichzeitig wird die Uebergabevorrichtung eingeschaltet und die Blutprobe durch die Schubstange 61 aus der Speicherscheibe in die Mischerscheibe befördert. Danach werden die Motoren für die Speicher- und Mischerscheibe wieder eingeschaltet.

Nach der Uebergabevorrichtung passiert die Speicherscheibe eine Gabel-Lichtschranke 152', welche auf der Platte 56 befestigt ist. Diese Lichtschranke wird bei jeder vollständigen Umdrehung der Speicherscheibe betätigt. Bei der Mischerscheibe dient die Gabel-Lichtschranke, die auf der Platte 19 befestigt und durch einen Stift 153 auslösbar ist, zum Zählen der Nuten auf der Scheibe. Nach der Uebergabevorrichtung 5 passiert die Mischscheibe eine weitere Gabel-Lichtschranke 152, welche auf der Platte 19 befestigt ist. Diese Lichtschranke wird bei jeder vollständigen Umdrehung der Mischerscheibe betätigt.

In der Blutentnahmeposition angekommen, wird der Motor für die Mischerscheibe ausgeschaltet. Die Motoren zum Herstellen einer Reibverbindung mit der Blutprobe und zum Drehen dieser um ihre Längsachse werden eingeschaltet. Gleichzeitig wird je nach Betriebsart bzw. Ausführungsform der Vorrichtung die Barcodelesevorrichtung 6 eingeschaltet. Diese liest einen allfällig vorhandenen Barcode. Wenn dieser nicht vorhanden ist (Barcodemode), wird die Reibungsverbindung mit der Blutprobe unterbrochen und beide Motoren werden wieder ausgeschaltet. Der Nadelträger wird nicht zur Mischerscheibe angehoben. Der Motor für die Mischerscheibe wird wieder eingeschaltet. Sie dreht weiter nach einer Auswurfposition 155, wo von einem dort angebrachten Elektromagnet 156 die Klemme geöffnet wird, so dass die nicht identifizierte Blutprobe aus der Nut fällt. Sie wird in dem Behälter 8 für nicht identifizierte Blutproben aufgefangen. Ist eine Blutprobe mit Barcode vorhanden und ist die Lesevorrichtung funktionstüchtig, so wird der Code gelesen und die Patientenidentifikation festgestellt und registriert. Die Barcodelesevorrichtung wird ausgeschaltet. Der Motor zum Drehen der Blutprobe wird ebenfalls ausgeschaltet. Nun hebt der Motor für den Nadelträger diesen an zur Blutprobe, wobei die Nadel die Gummikappe durchbohrt. Die erforderliche Blutmenge wird entnommen und dem Blutanalysator zugeführt, in welchem es analysiert wird. Nach der darauf folgenden Rückzugbewegung des Nadelträgers wird ein (nicht dargestellter) Wegschalter betätigt, der den Motor für die Mischerscheibe über die Steuerung wieder einschaltet. Das Zweiwegventil wird umgesteuert und die Nadel durchspült. Die Mischerscheibe dreht weiter nach der Auswurfposition 155, wo die Klemme von dem Elektromagneten 156 geöffnet wird. Die Blutprobe gleitet aus der Nut und in den Behälter 9 für identifizierte Blutproben. Bei der Ausführungsform ohne Barcodelesevorrichtung wird die Blutprobe in jedem einzelnen Fall verwertet, das heisst, jede Blutprobe wird angestochen.

Es kann vorkommen, dass in einem Notfall eine Blutprobe schnell, unter Umgehung der anderen Blutproben, analysiert werden muss. Für diesen Fall ist die Vorrichtung mit einem Schalter versehen, womit die besprochene Normal-Steuerung ausgeschaltet und eine Notfall-Steuerung eingeschaltet wird. Dieser Notfallbetrieb funktioniert folgendermassen: Sowohl in der Speicher- wie auch in der Mischerscheibe bleibt (auch im Normalbetrieb stets eine Position leer. Im Falle der dringend erforderlichen Untersuchung einer Notfall-Blutprobe, werden diese zwei Positionen verwendet. Dabei wird die Notfall-Blutprobe, nachdem sie von Hand gemischt wurde, in den Eingabeteil eingeschoben, aus welchem sie zur Speicherscheibe transportiert, von dieser übernommen und unverzüglich der Uebergabestelle zugeführt und dort von der Mischerscheibe übernommen wird. Von der Uebergabeposition wird sie ohne zusätzliche Mischungsumdrehung direkt der Stechstation zugeführt und dort ebenfalls bevorzugt bearbeitet. Anschliessend wird die benutzte Blutprobe über eine Notauswurfposition 158, wo ein Elektromagnet 159 die Klemme in der Mischerscheibe öffnet, in den dafür vorgesehenen, separat gehaltenen Behälter 10 abgelegt. Auf diese Weise werden Notfall-Blutproben und Routine-Blutproben auch physisch stets auseinander gehalten. Die evtl, vorhandene Barcode-Lesevorrichtung kann dabei normalerweise ausgeschaltet bleiben, weil bei Notfällen eine weitgehende organisatorische Erfassung, bis zur Codierung hin, in der Regel noch gar nicht vorliegt.

Nach dem Durchgang einer Notfall-Blutprobe wird die (wieder) freigewordene Position, sowohl auf der Mischer- sowie auch auf der Speicherscheibe für weitere Notfall-Blutproben freigehalten.

Die routinemässige Abarbeitung der Blutproben kann mit der diskutierten Vorrichtung in beliebig chargenweisem Vorgehen erfolgen. Da einerseits die Speicherscheibe mit bspw. 60 Nuten ständig in der Lage ist bis zu 59 Blutproben (eine freie Nut für den Notfallblutproben-Betrieb) aufzunehmen und in Bereitschaft zu halten und da andererseits die von der Speicherscheibe unabhängig angetriebene Mischerscheibe, mit der gleichen Aufnahmekapazität versehen, die Blutproben, dem Steuertakt des Analyseautomaten angepasst, entweder in zusammenhängender Folge oder schubweise ausgeben kann, ist eine verhältnismässig grosse innere Pufferkapazität vorhanden, die eine kaum ausschöpfbare äussere Flexibilität in Bezug auf die Organisation der Blutproben bewirkt. Die gesamte Pufferkapazität der Vorrichtung ist natürlich nicht auf die in diesem Beispiel genannten 118 Blutproben (2 mal 60 Nuten) beschränkt. Gemäss dem hier bekanntgegebenen Prinzip sind Speicherscheiben/Mischerscheiben-Paare mit erheblich mehr Aufnahmenuten möglich, die ggf. durch zwei Ladevorrichtungen gespiesen werden können, im Falle wo die Kadenz des Analsysautomaten sehr hoch sein sollte. Trotz der Möglichkeit einer nahtlosen Eingabesequenz, ist ein "Ueberspielen" des Ablaufes für Notfallblutproben stets zu jeder Zeit möglich. Dies alles ermöglicht eine Variationsbreite des Betriebsverfahrens derart, dass praktisch allen äusseren Erfordernissen bezüglich Organisation der Blutprobenanlieferung (Routine oder bei speziellen Aktionen), des Arbeitsablaufs der Analyse und (apparateseitig) denen des die Blutproben verarbeitenden Gerätes nachgekommen werden kann. Die vorgeschlagene Vorrichtung erfüllt somit alle Bedingungen, die an eine Schnittstelle für berechenbare hohe Automatisierung zur einem unberechenbaren Umfeld gefordert werden.

Ein von Hand eingegebener Blutprobebehälter wird in die Speicherscheibe übernommen, um entweder sofort (Notfallblutprobe) oder gemäss eingegebener Reihenfolge an eine Uebergabestelle befördert zu werden. Der Blutprobebehälter wird aus dem im Normalbetrieb schrittweise ablaufenden Speicherbetrieb an einen in der Regel ständig ablaufenden Mischbetrieb übergeben. Die Mindestmischzeit für die einzelnen Proben ist bestimmt. Nach Ablauf der vorgesehe nen Mischzeit wird (wahlweise) der Blutprobebehälter um die Behälterlängsachse rotiert, damit die Blutprobenidentifiaktion abgelesen werden kann. Dabei kann entschieden werden, ob die identifizierte Blutprobe verwertet oder eliminiert werden soll. Mit einer Blutentnahmevorrichtung wird dem Blutprobebehälter ein Teil des Blutes entnommen. Dazu wird die Mischerscheibe angehalten. Diese Entnahme geschieht durch den verschlossenen Behälter, indem mit einer Hohlnadel der flexible Behälterverschluss durchstochen wird. Anschliessend wird die Mischerscheibe wieder in Betrieb gesetzt. Nach jeder Entnahme wird die Hohlnadel mit einer Spüllösung ausgewaschen, wodurch sie für die nächste Entnahme wieder betriebsbereit ist.

Die Scheibentätigkeit wird überwacht. Jeder Durchgang einer Nute zur Aufnahme der Blutprobenbehälter und jede Scheibenumdrehung und jede Blutprobenbehälter-Uebergabe von der Speicher- zur Mischerscheibe werden elektronisch gespeichert und aus diesen gespeicherten Daten die Tätigkeit der beiden Antriebsmotoren für die Scheiben und der Antriebsmittel für die Lade-, Uebergabe-, Entnahme-und Auswerfevorrichtungen gesteuert. Ferner werden in einer weiteren Ausführungsform die abgespeicherten Daten einer Blutprobenidentifikation zur Gesamtsteuerung verwendet.

Die Steuereinheit übernimmt ferner Daten vom Analysegerät und von einer Handbedienung zur Steuerung der Ablaufgeschwindigkeit und des Taktes für die Blutprobenentnahme einerseits und für die (handausgelöste) Ueberschaltung auf einen Notfallblutproben-Analysebetrieb andererseits. Mit den Daten zur Auslösung des letztgenannten Betriebes werden vom Normalbetrieb separat gehaltene Betriebsabläufe aktiviert, aus welchen automatisch wieder auf den Normalbetrieb zurückgeschaltet wird.

Wenn bis anhin von einer Ausführungsform die Rede war, bei der lediglich eine Speicherscheibe und eine Mischerscheibe, also zwei Scheiben, zur Anwendung kamen, so sind Ausführungsformen mit mehr als einer Speicherscheibe dann wünschbar, wenn bspw. ein vorbestimmter Batch von Blutproben in die Vorrichtung aufgenommen werden soll, dessen Anzahl die Nutenzahl (minus 1) der Speicherscheibe übersteigt. In diesem Falle wird entsprechend der erfinderischen Vorschrift vorgegangen, nämlich einen Speicher- und einen Mischbetrieb für Blutproben zu realisieren, die unabhängig voneinander ablaufen und durch einen dem Speicherbetrieb zugeordneten Ladebetrieb für Blutprobenbehälter und durch einen dem Mischbetrieb zugeordneten Entnahmebetrieb für Blutproben, sowie durch einen Uebergabebetrieb zur Uebergabe der Blutprobenbehälter vom Speicher- in den Mischbetrieb oder zurück, der kurzzeitig den Speicherbetrieb und den Mischbetrieb miteinander verbindet wobei im Speicherbetrieb eine erste Speicherscheibe und eine zweite Speicherscheibe mit einer ersten Uebergabevorrichtung funktionell verbunden werden und die zweite Speicherscheibe mit der Mischerscheibe mit einer zweiten Uebergabevorrichtung funktionell verbunden werden. Der Lade- und der Entnahmebetrieb bleiben im wesentlichen gleichgeartet.

Es ist klar, dass mit der obigen Erweiterungsvorschrift weitere Nutenscheiben eingeführt werden können, wobei sich dies gleichwertig auf Lade- und/oder Mischerscheiben bezieht. Zwischen zwei Scheiben ist jeweils eine Uebergabevorrichtung angeordnet, die bei mehreren Scheiben dann gemeinsam einen kaskadierten Betrieb ermöglichen. Die einzelnen Scheiben sind unabhängig voneinander angetrieben, um die oben erwähnte Kommunikationsbedingung (S.5) erfülle zu können. Dabei weisen die "nicht-endständigen" Scheiben, im Gegensatz zu den "endständigen" achsial durchgehende Nuten auf. Allerdings können solche achsial durchgehende Nuten auch für die endständigen Scheiben vorgesehen sein.

Die Hinzufügung zusätzlicher Scheiben, bzw. die Zusammenfügung mehrerer Scheiben zu einem Diskstapel, lässt eine erweiterte verfahrenstechnische Flexibilität zu, die für einen kontinuierlichen Betrieb ausgenützt werden kann. Beispielsweise wird ein Stapelanteil zum Speichern benutzt (diese Scheiben bewegen sich nur für den Aufnahme-Uebergabebetrieb) der andere Stapelanteil wird zum Mischen benützt (diese Scheiben bewegen sich kontinuierlich). Nun besteht die Flexibilität darin, dass eine Speicherscheibe aus dem Speicherbetrieb in den Mischbetrieb versetzt wird oder analog dazu, dass eine Mischerscheibe aus dem Mischbetrieb in den Speicherbetrieb gebracht wird. Damit ist der jeweilige Stapelanteil Speichern/Mischen variabel und damit ist die Magazinierung von Blutproben eigentlich beliebig. So betrachtet, weist die Vorrichtung eine "innere" Pufferkapazität auf, die verfahrenstechnisch, also durch adäquaten Betrieb der Vorrichtung optimal genützt werden kann. Dabei kann der Stapel auch im Zweischeibenbetrieb arbeiten, in dem die inneren Scheiben still stehen und lediglich am Uebergabebetrieb beteiligt sind, während die endständigen Scheiben im Speicher- und im Mischbetrieb arbeiten. Sobald durch Zählung festgestellt wird, dass die Speicherscheibe am Ueberlaufen ist so beginnt die Nachbarscheibe in der Funktion als Speicherscheibe die Blutproben von der ersten Speicherscheibe zu übernehmen. Desgleichen kann eine Mischerscheibe angehalten werden und/oder in den Speicherbetrieb übergehen, so dies immerwie wünschbar ist. Dies ist ein weiterer Aspekt der oben erwähnten Flexibilität.

## Ansprüche

1. Vorrichtung zum Vorbereiten von Blutproben zur automatischen Analyse, gekennzeichnet durch mindestens eine Speicherscheibe (15) zur Speicherung der Blutproben und mindestens einer, unabhängig davon angetriebenen Mischerscheibe (17) zur Vorbereitung dieser Blutproben, welche Scheiben nebeneinander angeordnet sind und mit Nuten (42,43) zum Aufnehmen einer Blutprobe versehen sind, sowie durch je zwei Scheiben zugeordnete Uebergabevorrichtung/en (5) zum Uebergeben von Blutproben von eine Scheibe (15) an die andere Scheibe (17).

2. Vorrichtung zum Laden, Vorbereiten und Entnehmen von Blutproben zur automatischen Analyse gemäss Anspruch 1, gekennzeichnet durch die Anordnung einer Speicherscheibe (15) zur Speicherung der Blutproben und einer, unabhängig davon angetriebenen Mischerscheibe (17) zur Vorbereitung dieser Blutproben, welche Scheiben (15,17) auf der einander zugekehrten Seite mit radialen, einander komplementären Nuten (42,43) zum Aufnehmen einer Blutprobe versehen sind, weiterhin durch eine Ladevorrichtung (3) zum Laden von Blutproben in die Speicherscheibe (15), eine Uebergabevorrichtung (5) zum Uebergeben von Blutproben von der Speicherscheibe (15) an die Mischerscheibe (17), und eine Blutentnahmevorrichtung (7) für die Blutproben zum Zuführen des Blutes an eine Blutanalyseeinrichtung.

3. Vorrichtung nach Anspruch 1 oder 2, dadurch gekennzeichnet, dass eine Codelesevorrichtung (6) zum Identifizieren eines Codes auf der Blutprobe vorgesehen ist.

4. Vorrichtung nach den Ansprüchen 2 und 3, dadurch gekennzeichnet, dass die Codelesevorrichtung (6) im Bereich der Blutentnahmevorrichtung (7) angeordnet ist.

5. Vorrichtung nach Anspruch 2 oder 4, dadurch gekennzeichnet, dass die Ladevorrichtung (3) einen Schraubenspindelantrieb (81) aufweist, dessen Mutter (85) die Blutprobe in die korrespondierende Nut der alsdann stillstehende Speicherscheibe (15) befördert.

6. Vorrichtung nach Anspruch 5, dadurch gekennzeichnet, dass die Ladevorrichtung (3) mit einer Lichtschranke (95,96,97,97') versehen ist, die bei Betätigung durch die eingelegte Blutprobe über eine Steuerelektronik den Antriebsmotor für die Schraubenspindel (81) einschaltet.

7. Vorrichtung nach Anspruch 6, dadurch gekennzeichnet, dass die Ladevorrichtung (3) einen Endschalter (Zeichnung 7) aufweist, der durch die Mutter der Gewindespindel beim Verlassen der Ladevorrichtung betätigbar ist und welcher über die Steuerung den Antriebsmotor (33) für die Speicherscheibe (15) einschaltet.

8. Vorrichtung nach Anspruch 7, dadurch gekennzeichnet, dass die Ladevorrichtung (3) eine Schirmkappe (91) auf weist, welche die Ladevorrichtung nach dem Beginn des Ladevorgangs der Blutprobe gegen aussen abschliesst.

9. Vorrichtung nach einem der Ansprüche 2,4 oder 8, dadurch gekennzeichnet, dass die Uebergabevorrichtung (5) ein Kurbelantrieb (60) aufweist, die einen auf die Blutprobe in einer Nut der Speicherscheibe (15) gerichteten Stössel (61) betätigt, der die Blutprobe in die gegenüberliegende Nut der Mischerscheibe (17) befördert.

10. Vorrichtung nach Anspruch 9, dadurch gekennzeichnet, dass im Bereich der Uebergabevorrichtung (5) zwei Elektromagnete (70,71) angeordnet sind zum Oeffnen der für jede Nut der Speicherscheibe (15) und der Mischerscheibe (17) vorgesehenen Klemme (46,47) zum Sichern der Blutprobe in der jeweiligen Nut.

11. Vorrichtung nach einem der Ansprüche 2,4,8, oder 10, dadurch gekennzeichnet, dass die Blutentnahmevorrichtung (7) einen rotierenden Körper (129) aufweist, der an die Blutprobe heranfahrbar ist und mit dieser eine Reibungsverbindung hergestellt, wodurch die Blutprobe um ihre Längsachse in der Nut gedreht werden kann, und dass weiterhin ein Nadelträger (108) vorgesehen ist, der an die Blutprobe herangeführt, mit der daran angeordneten Nadel (114) den Verschluss (141) der Blutprobe durchbohren und Blut entnehmen kann.

12. Vorrichtung nach Anspruch 11, dadurch gekennzeichnet, dass eine Spülvorrichtung (145) vorgesehen ist, die nach dem Entnehmen der Blutprobe und nach dem Zurückfahren des Nadelträgers (108) ein Spülmittel in die Nadel (114) leitet, und dass in der Nadel (114) eine seitliche Bohrung (115) angebracht ist, durch welche das Spülmittel aus der Nadel austritt und aus dem Spühlraum (145) abgeführt werden kann.

13. Vorrichtung nach einem der Ansprüche 2,4,8,10 oder 12, dadurch gekennzeichnet, dass Sensormittel (150,150' 152,152') zur Ermittlung der Tätigkeit der Mischer- und der Speicherscheibe (15,17) vorgesehen sind.

14. Vorrichtung nach Anspruch 13, dadurch gekennzeichnet, dass erste Sensormittel (150,150') zur Zählung der Scheibennuten (42,43) und zweite Sensormittel (152,152') zur Zählung der Scheibenumdrehungen vorgesehen sind.

15. Vorrichtung nach Anspruch 14, dadurch gekennzeichnet, dass die Sensormittel (150,150',152,152') Lichtschranken sind.

16. Vorrichtung nach einem der Ansprüche 2,4,8,10,12 oder 15, dadurch gekennzeichnet,dass im Bereich der Auswurfstelle der Mischerscheibe (17) ein Elektromagnet (156) zum Oeffnen der Blutprobenklemme (47) vorgesehen ist.

Vorrichtung nach Anspruch 16, dadurch gekennzeichnet, dass eine Auswurfposition (155) für Notblutproben vor gesehen ist, die mit einem Elektromagneten (159) zum Oeffnen der Blutprobenklemme (47) versehen ist.

18. Vorrichtung nach Anspruch 17, dadurch gekennzeichnet, dass ein Behälter (8) zum Sammeln identifizierten Blutproben, ein Behälter (9) zum Sammeln von nicht identifizierbaren Blutproben und ein Behälter (10) für Notfallblutproben vorgesehen ist.

19. Vorrichtung nach einem der Ansprüche 1 bis 18, dadurch gekennzeichnet, dass die Speicher- und die Mischerscheibe (15,17) vertikal zur Unterlage angeordnet sind.

20. Vorrichtung nach einem der Ansprüche 1 bis 18, dadurch gekennzeichnet, dass die Speicher- und Mischerscheibe (15,17) geneigt zur Schwerkraftwirkung angeordnet sind.

21. Vorrichtung nach einem der Ansprüche 1 bis 18, dadurch gekennzeichnet, dass die Speicherscheibe (15) und die Mischerscheibe (17) im wesentlichen denselben Durchmesser aufweisen und koaxial angeordnet sind.

22. Vorrichtung nach Anspruch 21, dadurch gekennzeichnet, dass die Anzahl der Nuten in der Speicherscheibe (15) und die Anzahl der Nuten in der Mischerscheibe (17) gleich ist.

23. Verfahren zum Vorbereiten von Blutproben zur automatischen Analyse, gekennzeichnet durch je einen Speicher-und einen Mischbetrieb für Blutproben, die unabhängig voneinander ablaufen und durch einen dem Speicherbetrieb zugeordneten Ladebetrieb für Blutprobenbehälter und durch einen dem Mischbetrieb zugeordneten Entnahmebetrieb für Blutproben, sowie durch einen Uebergabebetrieb zur Uebergabe der Blutprobenbehälter vom Speicherbetrieb in den Mischbetrieb oder zurück, der kurzzeitig den Speicherbetrieb und den Mischbetrieb miteinander verbindet.

24. Verfahren nach Anspruch 23, dadurch gekennzeichnet, dass Blutprobenbehälter in eine Speicherscheibe mit Aufnahmenuten für den Behälter geladen werden, die Behälter in eingegebener Reihenfolge an eine Uebergabestelle befördert, um vom Speicherbetrieb an den Mischbetrieb übergeben zu werden, in dem die Behälter von den Aufnahmenuten in der Speicherscheibe in solche in der Mischerscheibe gestossen werden, dass nach einer Mindestmischzeit mittels einer Blutentnahmevorrichtung dem Blutprobebehälter ein Teil des Blutes entnommen wird.

25. Verfahren nach Anspruch 24, dadurch gekennzeichnet, dass vor der Entnahme einer Blutprobe der Blutprobebehälter um die Behälterlängsachse rotiert und gleichzeitig die Blutprobenidentifiaktion abgelesen wird.

26. Verfahren nach Anspruch 25, dadurch gekennzeichnet, dass die Blutprobenidentifikation zur Entscheidung benützt wird, ob die identifizierte Blutprobe verwertet oder eliminiert werden soll.

27. Verfahren nach einem der Ansprüche 24 bis 26, dadurch gekennzeichnet, dass sowohl in der Speicher- wie auch in der Mischerscheibe (auch im Normalbetrieb) stets eine Position leer bleibt.

28. Verfahren nach Anspruch 27, dadurch gekennzeichnet, dass im Falle einer dringend erforderlichen Untersuchung einer Notfall-Blutprobe, diese zwei Positionen folgendermassen verwendet werden, der Normalbetrieb wird ausgeschaltet, die freie Position in der Speicherscheibe wird zur Ladestelle und die freie Position der Mischerscheibe zur Uebergabestelle bewegt, der in die freie Position in der Speicherscheibe geladene Blutprobenbehälter wird unverzüglich zur Uebergabestelle befördert, von dieser übernommen und in die freie Position in der Mischerscheibe übernommen und von der Uebergabeposition ohne zusätzliche Mischungsdrehung direkt der Stechstation zugeführt.

29. Verfahren nach einem der Ansprüche 23 bis 28, dadurch gekennzeichnet, dass die benutzten Blutproben in verschiedene separat gehaltenen Behälter abgelegt werden, um sie auch physisch auseinander zu halten.

30. Verfahren nach einem der Ansprüche 23 bis 28, dadurch gekennzeichnet, dass nach jeder Blutentnahme aus einem Blutprobenbehälter die mit dem Blut in Kontakt geratenen Teile der Entnahmevorrichtung mit einer Spüllösung gewaschen werden.

31. Verfahren nach einem der Ansprüche 23 bis 30, dadurch gekennzeichnet, dass die Scheibentätigkeit überwacht wird, indem jeder Durchgang einer Nute zur Aufnahme der Blutprobenehälter und jede Scheibenumdrehung und jede Blutprobenbehälter-Uebergabe von der Speicher- zur Mischerscheibe elektronisch gespeichert wird und aus diesen gespeicherten Daten die Tätigkeit der beiden Antriebsmotoren für die Scheiben und der Antriebsmittel für die Lade-, Uebergabe-, Entnahme-, und Auswerfevorrichtungen gesteuert werden.

32. Verfahren nach Anspruch 23 bis 31, gekennzeichnet durch die Einfügung zusätzlicher Scheiben, bzw. die Zusammenfügung mehrerer Scheiben zu einem Diskstapel, wobei ein Stapelteil zum Speichern benützt und der andere Stapelteil zum Mischen benützt wird.

33. Verfahren nach Anspruch 32, dadurch gekennzeichnet, dass eine Speicherscheibe aus dem Speicherbetrieb in den Mischbetrieb versetzt wird oder dass eine Mischerscheibe aus dem Mischbetrieb in den Speicherbetrieb gebracht wird.

34. Verfahren nach Anspruch 33, dadurch gekennzeichnet, dass der Stapelanteil für den Speicher- und für den Mischbetrieb variabel gehalten wird, um die Magazinierung von Blutproben dem Blutprobenangebot anzupassen.

35. Verfahren nach Anspruch 32 bis 34, dadurch gekennzeichnet, dass der Scheibenstapel in einem Zweischeibenbetrieb gesteuert wird, in dem die inneren Scheiben still stehen und lediglich am Uebergabebetrieb beteiligt sind, während die endständigen Scheiben im Speicher- und im Mischbetrieb arbeiten.

36. Verfahren nach Anspruch 32 bis 35, dadurch gekennzeichnet, dass nach Auffüllung der Speicherscheibe (Nutenzahl minus 1) die Nachbarscheibe in Richtung Mischerscheibe/n in der Funktion als Speicherscheibe die Blutproben von der ersten Speicherscheibe übernehmen.

FIG. 1

FIG. 2

FIG. 3

FIG. 4

FIG. 5

FIG. 6

FIG. 7

FIG. 8

FIG. 9

FIG. 10